# EUROPEAN PATENT SPECIFICATION

(11) **EP 4 193 947 B1**
(45) Date of publication and mention of the grant of the patent: **13.11.2024**
(21) Application number: 22212474.5
(22) Date of filing: 09.12.2022
(51) Int. Cl.: A61B 18/14, A61B 5/00, A61B 18/00, A61B 5/283, A61B 18/16

(54) **BASKET CATHETER WITH ELECTRICALLY-CONNECTED SPINES FORMING A DISTRIBUTED ELECTRODE**
KORBKATHETER MIT ELEKTRISCH VERBUNDENEN DORNEN ZUR FORMUNG EINER VERTEILTEN ELEKTRODE
CATHÉTER PANIER AVEC DES COLONNES RELIÉES ÉLECTRIQUEMENT FORMANT UNE ÉLECTRODE DISTRIBUÉE

(30) Priority: 10.12.2021 US 202117548278
(43) Date of publication of application: 14.06.2023
(73) Proprietor: Biosense Webster (Israel) Ltd., 2066717 Yokneam (IL)
(72) Inventor: GOVARI, Assaf, 2066717 Yokneam (IL); BEECKLER, Christopher Thomas, Irvine, 92618 (US)
(74) Representative: Carpmaels & Ransford LLP

(56) References cited:
- US-A1- 2013 090 651
- US-A1- 2021 187 241
- US-A1- 2021 282 847
- US-A1- 2021 307 815

## Description

### FIELD OF THE INVENTION

The present invention relates generally to medical probes, and particularly to multi-electrode ablation catheters.

### BACKGROUND OF THE INVENTION

Cardiac arrythmias are commonly treated by ablation of myocardial tissue in order to block arrhythmogenic electrical pathways. For this purpose, a catheter is inserted through the patient's vascular system into a chamber of the heart, and an electrode or electrodes at the distal end of the catheter are brought into contact with the tissue that is to be ablated with a suitable energy. In some cases, high-power radio-frequency (RF) electrical energy is applied to the electrodes in order to ablate the tissue thermally. Alternatively, high-voltage pulses may be applied to the electrodes in order to ablate the tissue by irreversible electroporation (IRE) also known as pulse field ablation.

Some ablation procedures use basket catheters, in which multiple electrodes are arrayed along the spines of an expandable basket assembly at the distal end of the catheter. The spines bend outward to form a basket-like shape and contact tissue within a body cavity. For example, U.S. Patent Application Publication 2020/0289197 describes devices and methods for electroporation ablation therapy, with the device including a set of spines coupled to a catheter for medical ablation therapy. Each spine of the set of spines may include a set of electrodes formed on that spine. The set of spines may be configured for translation to transition between a first configuration and a second configuration.

US 2013/0090651 A1 provides systems for nerve modulation through the wall of a blood vessel. An example system for nerve modulation may include an elongate member extending along a central elongate axis and having a proximal end and a distal end. The elongate member may have a radially expandable member disposed proximate the distal end. A tubular sheath may be cooperatively engaged with the expandable member such that the expandable member is collapsed when in the sheath and can expand when moved distally relative to and past a distal end of the sheath. The expandable member may include a plurality of electrodes and a plurality of spacer struts.

### SUMMARY OF THE INVENTION

The invention provides a medical probe according to claim 1. Embodiments are provided by the dependent claims.

Certain methods which do not form part of the invention, are described with reference to the medical probe of the present invention. Whilst no claim is directed to these methods *per se,* the medical probe is capable of being used and is intended to be used in such methods. For instance, there is provided a method including inserting, into a cavity of an organ of a patient, a medical probe including a shaft and a basket assembly connected at a distal end of the shaft. The basket assembly includes (i) multiple electrically-conductive spines that are electrically-connected to one another so as to form a distributed electrode, and (ii) a plurality of spine mounted electrodes disposed along the spines. Using the plurality of spine mounted electrodes, electrical activity is sensed in the cavity while the distributed electrode is prevented from indenting tissue in the cavity.

There is additionally provided a method for producing a medical probe. The method includes producing a basket assembly, including multiple electrically-conductive spines that are electrically-connected to one another so as to form a distributed electrode, and a plurality of spine mounted electrodes, which are disposed along the spines and are configured to (i) sense electrical activity in the cavity, and (ii) prevent the distributed electrode from indenting tissue in the cavity. The basket assembly is connected to a shaft configured for insertion into a cavity of an organ of a patient.

The present invention will be more fully understood from the following detailed description of the embodiments thereof, taken together with the drawings in which:

### BRIEF DESCRIPTION OF THE DRAWINGS

Fig. 1 is a schematic pictorial illustration of a catheter-based electrophysiological procedure utilizing an ablation system comprising a basket catheter, in accordance with an embodiment of the present invention;
Fig. 2 is a schematic side view of the basket catheter assembly of Fig. 1, in accordance with an embodiment of the invention; and
Fig. 3 is a schematic pictorial illustration of the basket catheter assembly of Fig. 1 positioned to ablate an ostium of a pulmonary vein, in accordance with an embodiment of the invention.
Fig. 4 illustrates another example of the basket catheter.

### DETAILED DESCRIPTION OF EMBODIMENTS

### OVERVIEW

A multi-electrode cardiac catheter typically comprises a distal-end assembly disposed with multiple electrodes. For example, a basket catheter typically comprises an expandable frame of spines which is coupled to the distal end of a shaft for insertion into a cavity of an organ of a patient.

Basket catheters are useful to perform ablation procedures rapidly and efficiently, since the spines of the basket catheter (and thus the electrodes on the spines) are able to contact and ablate the tissue at multiple locations concurrently. These spine mounted electrodes, however, can indent the tissue during the procedure, which can lead to local overheating, resulting in charring and/or other trauma, in particular with RF ablation. The use of electrodes having smooth, rounded profiles can be helpful in mitigating these effects, but by itself its use does not eliminate the problems of tissue damage.

Embodiments of the present invention that are described herein provide a basket catheter with a distributed electrode configured to apply ablative signal safely. The distributed electrode is made of the basket spines wired to deliver RF and/or IRE in unipolar mode against an indifferent electrode (e.g., a back patch). In addition, multiple spine mounted electrodes are disposed on the spines, for performing electrophysiological (EP) sensing or ablating. The spines are electrically-conductive and are typically electrically uninsulated (exposed) except for small regions around each of the spine mounted electrodes. The spines are electrically-connected to one another (e.g., by contacting each other at the distal end of the basket and/or shorting connecting wires together), so the spines together form the distributed electrode (e.g., for use in RF ablation in a unipolar mode, but other possible usages are described below). The insulation typically covers short sections of the spines, just enough to prevent sparking between a spine mounted electrode and a spine, or between electrodes on adjacent spines. In some configurations, where the spines are configured as a single electrode, the mounted electrode (or electrodes) can be the indifferent electrode(s).

The distributed electrode spreads the ablation (RF or IRE) energy in a way that can reduce thermal hazard to tissue, such as charring. Typically, the spine mounted electrodes are made thicker than the spines, causing the basket assembly to act as a mechanical barrier that prevents the distributed electrode from burying into the tissue (e.g., identing the tissue). In other words, the thick spine mounted electrodes act as "bumpers" to prevent the spines (the distributed electrode) from pressing too strongly against the tissue during the RF ablation. At the same time, the spine mounted electrodes can be used to acquire electrograms to verify an efficacy of the ablation, such as in an arrhythmia-treatment procedure of pulmonary vein isolation.

### SYSTEM DESCRIPTION

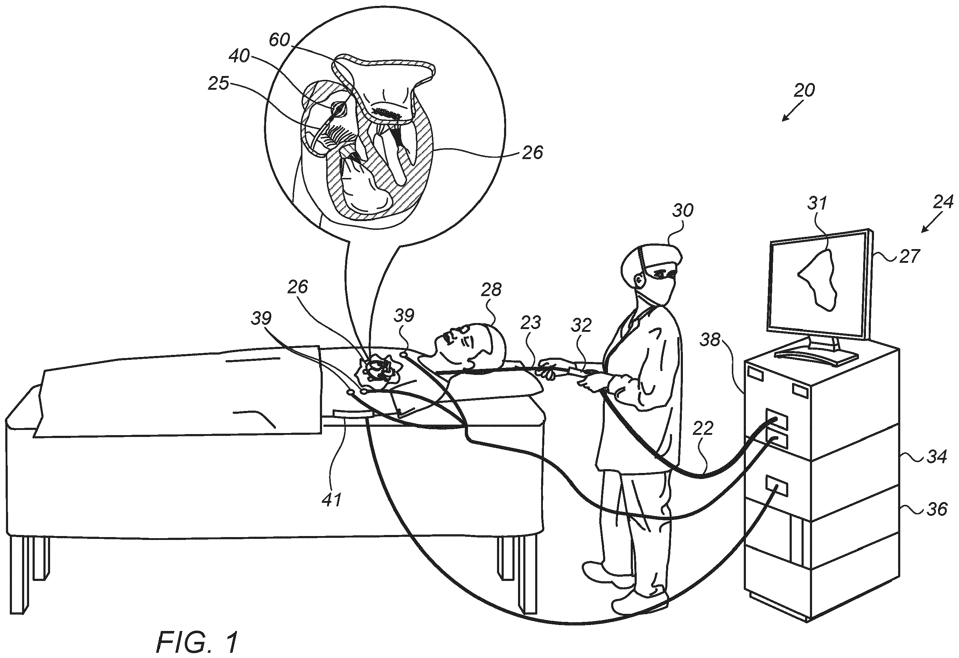

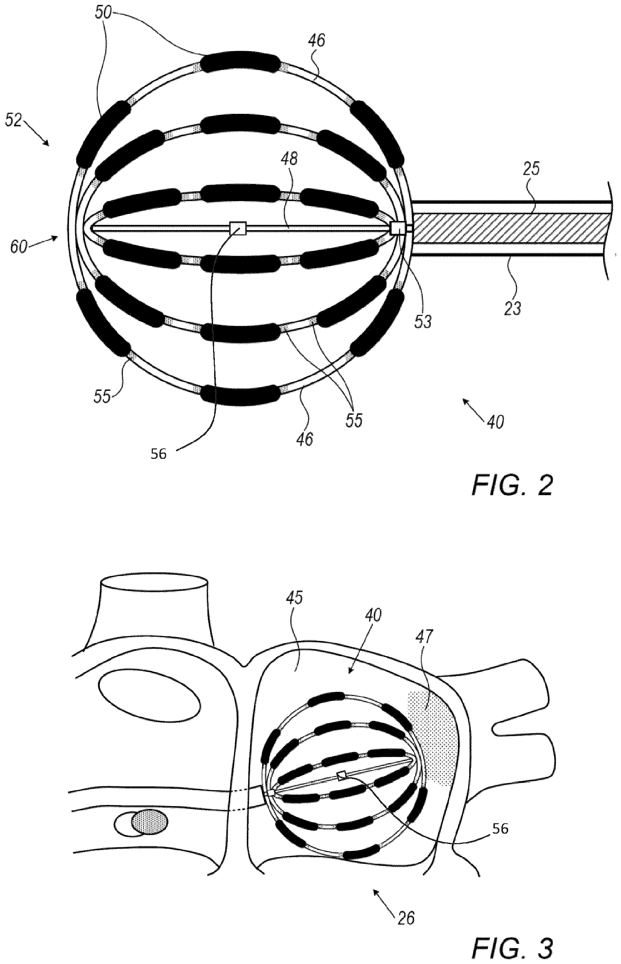

Fig. 1 is a schematic pictorial illustration of a catheter-based electrophysiological procedure utilizing an ablation system 20 comprising a basket catheter 22, in accordance with an embodiment of the present invention. Elements of system 20 may be based on components of the CARTO^{®} system, produced by Biosense Webster, Inc. (Irvine, California). Spines of a basket assembly 40 connected at the distal end of catheter 22 contact each other at a distal end of the basket, so the spines form a distributed electrode 60, as further described in Fig. 2.

In one embodiment, distributed electrode 60 is used for RF ablation. In another embodiment, distributed electrode 60 may be used as a reference electrode for electrophysiological mapping done using the mounted electrodes 50. In yet another embodiment, distributed electrode 60 may serve as a shared reference electrode during IRE ablation by spine mounted electrodes 50. For all the above purposes, the spines are electrically shortcircuited and connected to a single wire (not shown) running in shaft 25 to a console 24. Alternatively, each spine may be individually wired and all wires are electrically shorted together within ablation system 20. Spine mounted electrodes 50 are individually wired and can each be used separately.

A physician 30 navigates a catheter 22 through the vascular system of a patient 28 into a chamber of a heart 26 of the patient, and then deploys basket catheter assembly 40. The proximal end of basket catheter assembly 40 is connected to the distal end of a shaft 25, which physician 30 steers using a manipulator 32 near the proximal end of catheter 22. Basket catheter assembly 40 is inserted in a collapsed configuration through a tubular sheath 23, which passes through the vascular system of patient 28 into the heart chamber where the ablation procedure is to be performed.

Once inserted into the heart chamber, basket catheter assembly 40 is deployed from the tubular sheath and allowed to expand within the chamber. Catheter 22 is connected at its proximal end to a control console 24. A display 27 on console 24 may present a map 31 or other image of the heart chamber with an icon showing the location of basket catheter assembly 40 in order to assist physician 30 to position the basket assembly at the target location for the ablation procedure.

In one embodiment, once basket catheter assembly 40 is properly deployed and positioned in heart 26, physician 30 actuates an electrical signal generator 38 in console 24 to apply electrical energy (such as RF waveforms or high voltage IRE pulses) to distributed electrode 60 in a unipolar mode, i.e., between the spine mounted electrodes on basket catheter assembly 40 and a separate external common electrode, for example a conductive back patch 41 which is applied to the patient's skin. During the ablation procedure, an irrigation pump 34 delivers an irrigation fluid, such as saline solution, through shaft 25 to basket catheter assembly 40. In this embodiment, spine mounted electrodes 50 can be used as electro-physiologically sensing electrodes to acquire electrograms, as discussed in Fig. 3.

Typically, catheter 22 comprises one or more position sensors (not shown in the figures), which output position signals that are indicative of the position (location and orientation) of basket catheter assembly 40. For example, basket assembly 40 may incorporate one or more magnetic sensors which output electrical signals in response to an applied magnetic field. Processor 36 receives and processes the signals in order to find the location and orientation coordinates of basket catheter assembly 40, using techniques that are known in the art and are implemented, for example, in the above-mentioned Carto system. Alternatively or additionally, system 20 may apply other position-sensing technologies in order to find the coordinates of basket catheter assembly 40. For example, processor 36 may sense the impedances between the electrodes on basket catheter assembly 40 and body surface electrodes 39, which are applied to the chest of patient 28, and may convert the impedances into location coordinates using techniques that are likewise known in the art. In any case, processor 36 uses the coordinates to display the location of basket catheter assembly 40 on map 31.

Alternatively, catheter 22 and the ablation techniques that are described herein may be used without the benefit of position sensing. In such embodiments, for example, fluoroscopy and/or other imaging techniques may be used to ascertain the location of basket catheter assembly 40 in heart 26.

The system configuration that is shown in Fig. 1 is presented by way of example for conceptual clarity in understanding the operation of embodiments of the present invention. For the sake of simplicity, Fig. 1 shows only the elements of system 20 that are specifically related to basket catheter assembly 40 and ablation procedures using the basket assembly.

### BASKET CATHETER WITH SPINES CONTACTING EACH OTHER TO FORM A DISTRIBUTED ELECTRODE

Fig. 2 is a schematic side view of basket catheter assembly 40 of Fig. 1, in accordance with an embodiment of the invention. Basket catheter assembly 40 is drawn in expanded state outside sheath 23.

As seen, basket catheter assembly 40 has a distal end 52 and a proximal end 53, which is connected to a distal end of shaft 25. The basket catheter assembly comprises multiple spines 46, whose proximal ends are conjoined at proximal end 53, and whose distal ends are conjoined at distal end 52. Multiple thick rounded and spine mounted electrodes 50 are disposed externally on each of spines 46.

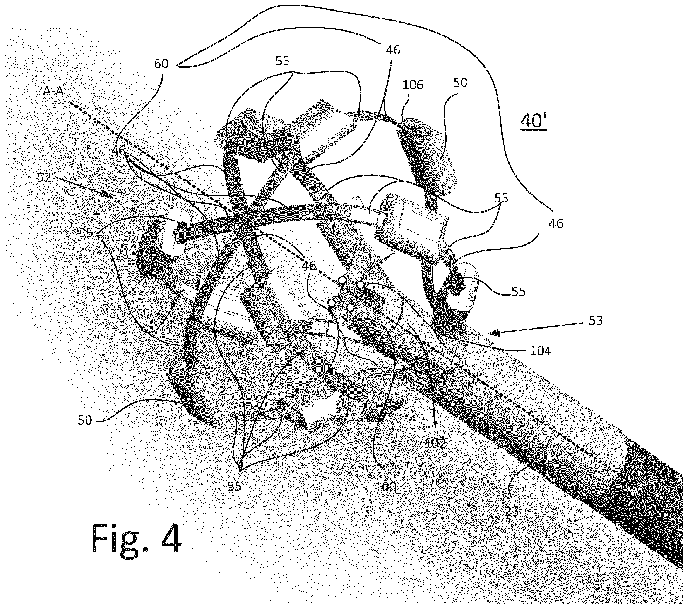

Spines 46 are electrically conducting and are uninsulated so that these portions of spines 46 (as indicated by lead lines in Fig. 4) are exposed to the ambient environment (or biological tissues and fluids). It should be noted that the spines 46 are provided with small insulated regions 55 around each of electrodes 50. Electrically conducting spines 46 contact each other at the distal end 52 of the basket, so the exposed surface of the spines form distributed electrode 60. Because of the spines 46 being electrically connected together to form the distributed electrode 60, distributed electrode 60 can be thought of as a single large electrode.

In the shown expanded state, spines 46 bow radially outward. In the collapsed state (not shown), spines 46 are straight and aligned parallel to a longitudinal axis of shaft 25 to facilitate insertion of basket catheter assembly 40 into heart 26.

In one embodiment, spines 46 are produced such that the stable state of basket catheter assembly 40 is the collapsed state. In this case, when basket catheter assembly 40 is pushed out of the sheath, it is expanded by drawing an actuator wire or rod (48 in Fig. 2) in the proximal direction through shaft 25. Releasing the actuator rod or puller 48 wire allows basket catheter assembly 40 to collapse back to its original state. A reference electrode 56 can be provided on the actuator rod 48 so that far field tissue generated signals can be collected with the signals collected by the spine mounted electrodes 50 and used for noise reduction or cancellation.

In another embodiment, spines 46 are produced such that the stable state of basket catheter assembly 40 is the expanded state of Fig. 2. In this case, basket catheter assembly 40 opens out into the expanded stated when it is pushed out of the sheath, and the puller wire may be replaced by a flexible pusher rod 48 for straightening spines 46 before withdrawing the basket catheter assembly back into the sheath.

Fig. 2 is illustrated in a schematic manner for ease of understanding. It is noted that, the actual shape of spine mounted electrodes 50 differs than shown. As another example, in some embodiments irrigation outlets in spines 46 allow irrigation fluid flowing within the spines to exit and irrigate tissue in the vicinity of electrodes 50.

In the embodiment shown in Figs. 1 and 2 the spines are electrically in contact one with the other at a distal end of the basket catheter assembly. In another embodiment, the spines are electrically in contact one with the other at a proximal end of basket catheter assembly.

Fig. 3 is a schematic pictorial illustration of basket catheter assembly 40 of Fig. 1 positioned to ablate an ostium 47 of a pulmonary vein, in accordance with an embodiment of the invention. In this exemplary procedure, the catheter is first introduced to the right atrium (RA) via the inferior vena cava (IVC), where it passes through a puncture in the fossa ovalis of the interatrial septum in order to reach the left atrium (LA) 45. Catheter assembly 40 is to be distally pressed against ostium 47, with distributed electrode 60 conveying RF energy to ostium 47 tissue. At the same time spine mounted electrodes 50, due to the electrodes being large and protruding outwards relative the spines of electrode 60, prevent the RF-delivering spines from indenting ostium 47 tissue and causing mechanical or thermal damage to tissue during the RF ablation.

In this embodiment, when basket catheter assembly is pressed against tissue, the spine mounted electrodes prevent indentation of tissue by the distributed electrode by being surface mounted on the spines and having a minimal given thickness in the radial direction (e.g., mounted in a spatially biased way to be mostly external to the spines) .

Bulky electrodes on a basket catheter, which are asymmetrically mounted on spines with most of the electrode thickness protruding radially outwards the spines, are described in U.S. Patent application 17/371,008, titled, "BIASED ELECTRODES FOR IMPROVED TISSUE CONTACT AND CURRENT DELIVERY," filed July 8th, 2021.

When pressed against ostium 47 tissue, spine mounted electrodes 50 acquire electrophysiological signals (e.g., electrograms), and processor 36 analyzes these signals to verify that arrhythmogenic electrical pathways are ablated over an entire circumference of ostium 47, so an arrhythmia caused there is fully treated by the ablation.

Fig. 4 shows an alternative end effector 40' extending along longitudinal axis A-A defined by the tubular shaft 23. In this example, the electrodes 50 have a scarab like profile. Specifically, electrode 50 is provided with a hole 106 extending through the electrode 50. The through hole 106 allows the spine 46 to be inserted into through hole 106 and secured by a suitable technique (e.g., adhesives, mechanical tabs or via welding) to the spine 46 where insulation material 55 is formed over the exposed surface of spine 46 where the spine mounted electrodes 50 are located. An irrigation tube 100 with irrigation holes 104 can be provided to allow for irrigation fluid to be dispersed around the single distributed electrode 60 (formed by the electrically shorted spines 46) during ablation. A central electrode 102 can be provided to act as a reference electrode for noise reduction of ECG signals or as a return (or indifferent) electrode for ablation purposes.

As shown in the drawings herein, distributed electrode 60 (formed by spines 46 electrically shorted together) can be practiced in a method to deliver ablative energy in various modes. The ablation modes allow for delivering of ablative energy (RF or IRE) to either one or both of the distributed electrode 60 and at least one of the spine mounted electrodes 50. In one mode, ablation energy (RF or IRE) can be provided to at least one of the spine mounted electrodes 50 and configuring the distributed electrode 60 to act as a return electrode for the ablative energy provided to the spine electrode(s) 50. In another mode, the system can be configured for delivering ablative energy to distributed electrode 60 and configuring at least one of the spine mounted electrodes 50 as return electrodes. In yet a third mode, ablative energy can be provided in a unipolar mode to the distributed electrode 60 or at least one of the spine mounted electrodes 50 with the external body electrodes 41 acting as the return electrode.

To summarize, the single distributed electrode 60 can operate in the following modes: (1) bipolar ablation mode with one or more of the spine mounted electrodes 50 being the return or indifferent electrodes; (2) bipolar ablation mode with the central electrode (56 or 102) as the return or indifferent electrode; or (3) unipolar ablation mode with the indifferent electrode being the known electrode patches 41.

On the other hand, spine mounted electrodes 50 can be used to: (1) map or collect electrical signal emanating from biological tissues; (2) deliver ablative energy in bipolar mode with the distributed electrode 60 acting as the return electrode; (3) deliver ablative energy in bipolar mode with the central electrode 56 or 102 as the return electrode; or (4) deliver ablative energy in unipolar mode with the body patch 41 as the indifferent electrode.

Moreover, it is noted that the distributed electrode 60 can deliver RF energy while the spine mounted electrodes can deliver IRE energy. This can be reversed in that the distributed electrode 60 can deliver IRE energy while the spine mounted electrodes 50 can deliver RF energy.

The spine mounted electrodes 50 have the ability to deliver energy independently to a small subset of electrodes 50, sequentially or simultaneously whereas the distributed electrode 60 can only deliver ablation energy simultaneously to the entire structure. In one exemplary method, an operator can transport the end effector 40 or 40' into the target organ, map the organ to determine a targeted site for ablation, deliver RF energy (or IRE energy) to the distributed electrode 60, map the resulting tissues via the spine mounted electrodes 50 to determine if ablated site has been electrically isolated and depending the size of the area needing further ablation, some of the spine mounted electrode can be used to deliver IRE energy (or RF energy) from a small subset of spine mounted electrodes to ensure a complete isolation of the targeted site. Techniques for application of the pulse fields are provided in US20210307815. Although the embodiments described herein mainly address cardiac applications, the methods and systems described herein can also be used in other catheter-based applications, such as in performing ablation inside other organs, e.g., inside a bladder or in the renal artery outside of the kidney. It will thus be appreciated that the embodiments described above are cited by way of example, and that the present invention is not limited to what has been particularly shown and described hereinabove. Rather, the scope of the present invention is defined by the appended claims.

## Claims

1. A medical probe, comprising:
a shaft (25) defining a longitudinal axis, the shaft (25) configured for insertion into a cavity of an organ of a patient;
a basket assembly (40), which is connected at a distal end of the shaft (25) and extending along the longitudinal axis, the basket (40) comprises:
multiple electrically-conductive spines (46) that are electrically-connected to one another so as to form a distributed electrode;
**characterized in that** it comprises
a plurality of spine mounted electrodes (50), which are disposed along the spines (46), are electrically insulated from the spines (46), and are configured to (i) sense electrical activity in the cavity, and (ii) prevent the distributed electrode from indenting tissue in the cavity.

2. The medical probe according to claim 1, wherein the spines (46) are electrically-connected to one another at a distal end of the basket assembly (40).

3. The medical probe according to claim 1, wherein the spines (46) are electrically-connected to one another at a proximal end of the basket assembly (40).

4. The medical probe according to claim 1, wherein the distributed electrode is configured to apply an ablation signal.

5. The medical probe according to claim 1, wherein the spine mounted electrodes (50) protrude radially outward from the spines (46) and away from the longitudinal axis so as to prevent the distributed electrode from indenting the tissue.

6. The medical probe according to claim 5, wherein the spine mounted electrodes (50) are surface-mounted on the spines (46) and have a given thickness external to the longitudinal axis and protruding externally to the spines.

7. The medical probe of claim 1, further comprising basket actuator (48) extending from the shaft (25) to a distal end of the basket (40) and connected to the spines (46) so that movement of the actuator (48) along the longitudinal axis causes a change in shape of the basket (40) .

8. The medical probe of claim 1, further comprising an irrigation member (100) extending from the distal portion of the shaft (25).

9. The end effector of claim 1, further comprising a reference electrode disposed on a member extending from a tubular member disposed within the spines and about the longitudinal axis.

## Patentansprüche

1. Medizinische Sonde, umfassend:
einen Schaft (25), der eine Längsachse definiert, wobei der Schaft (25) für eine Einführung in einen Hohlraum eines Organs eines Patienten konfiguriert ist;
eine Korbanordnung (40), die an einem distalen Ende des Schafts (25) verbunden ist und sich entlang der Längsachse erstreckt, wobei der Korb (40) umfasst:
mehrere elektrisch leitfähige Stacheln (46), die miteinander elektrisch verbunden sind, um eine verteilte Elektrode auszubilden;
**dadurch gekennzeichnet, dass** es umfasst
eine Vielzahl von an den Elektroden (50) montierten Stacheln, die entlang der Stacheln (46) eingerichtet sind, die von den Stacheln (46) elektrisch isoliert sind, und konfiguriert sind, um (i) elektrische Aktivität in dem Hohlraum zu erfassen, und (ii) zu verhindern, dass die verteilte Elektrode das Gewebe in dem Hohlraum eindrückt.

2. Medizinische Sonde nach Anspruch 1, wobei die Stacheln (46) an einem distalen Ende der Korbanordnung (40) miteinander elektrisch verbunden sind.

3. Medizinische Sonde nach Anspruch 1, wobei die Stacheln (46) an einem proximalen Ende der Korbanordnung (40) miteinander elektrisch verbunden sind.

4. Medizinische Sonde nach Anspruch 1, wobei die verteilte Elektrode konfiguriert ist, um ein Ablationssignal anzulegen.

5. Medizinische Sonde nach Anspruch 1, wobei die an den Stacheln montierten Elektroden (50) radial nach außen von den Stacheln (46) und von der Längsachse weg vorstehen, um zu verhindern, dass die verteilten Elektroden das Gewebe eindrücken.

6. Medizinische Sonde nach Anspruch 5, wobei die an den Stacheln montierten Elektroden (50) auf den Stacheln (46) oberflächenmontiert sind und eine gegebene Dicke außerhalb der Längsachse aufweisen und außerhalb der Stacheln hervorstehen.

7. Medizinische Sonde nach Anspruch 1, ferner umfassend einen Korbaktuator (48), der sich von dem Schaft (25) zu einem distalen Ende des Korbs (40) erstreckt und mit den Stacheln (46) verbunden ist, so dass eine Bewegung des Aktuators (48) entlang der Längsachse eine Formänderung des Korbs (40) bewirkt.

8. Medizinische Sonde nach Anspruch 1, ferner umfassend ein Spülelement (100), das sich von dem distalen Abschnitt des Schafts (25) aus erstreckt.

9. Endeffektor nach Anspruch 1, ferner umfassend eine Referenzelektrode, die auf einem Element eingerichtet ist, das sich von einem röhrenförmigen Element erstreckt, das innerhalb der Stacheln und um die Längsachse herum eingerichtet ist.

## Revendications

1. Sonde médicale, comprenant :
une tige (25) définissant un axe longitudinal, la tige (25) étant configurée pour être insérée dans une cavité d'un organe d'un patient ;
un ensemble panier (40), qui est relié à une extrémité distale de la tige (25) et qui s'étend le long de l'axe longitudinal, le panier (40) comprend :
de multiples colonnes électriquement conductrices (46) qui sont électriquement connectées les unes aux autres de manière à former une électrode distribuée ;
**caractérisée en ce qu'**elle comprend
une pluralité d'électrodes (50) montées sur des colonnes, disposées le long des colonnes (46), isolées électriquement des colonnes (46) et configurées pour (i) détecter une activité électrique dans la cavité et (ii) empêcher l'électrode distribuée de pénétrer dans des tissus de la cavité.

2. Sonde médicale selon la revendication 1, dans laquelle les colonnes (46) sont électriquement connectées les unes aux autres au niveau d'une extrémité distale de l'ensemble panier (40).

3. Sonde médicale selon la revendication 1, dans laquelle les colonnes (46) sont électriquement connectées les unes aux autres au niveau d'une extrémité proximale de l'ensemble panier (40).

4. Sonde médicale selon la revendication 1, dans laquelle l'électrode distribuée est configurée pour appliquer un signal d'ablation.

5. Sonde médicale selon la revendication 1, dans laquelle les électrodes (50) montées sur les colonnes font saillie radialement vers l'extérieur des colonnes (46) et loin de l'axe longitudinal de manière à empêcher l'électrode distribuée de pénétrer dans les tissus.

6. Sonde médicale selon la revendication 5, dans laquelle les électrodes (50) montées sur les colonnes sont montées en surface sur les colonnes (46) et ont une épaisseur donnée à l'extérieur de l'axe longitudinal et faisant saillie à l'extérieur des colonnes.

7. Sonde médicale selon la revendication 1, comprenant en outre un actionneur de panier (48) s'étendant depuis la tige (25) jusqu'à une extrémité distale du panier (40) et relié aux colonnes (46) de sorte qu'un mouvement de l'actionneur (48) le long de l'axe longitudinal provoque un changement de forme du panier (40).

8. Sonde médicale selon la revendication 1, comprenant en outre un élément d'irrigation (100) s'étendant depuis la partie distale de la tige (25).

9. Effecteur selon la revendication 1, comprenant en outre une électrode de référence disposée sur un élément s'étendant depuis un élément tubulaire disposé à l'intérieur des colonnes et autour de l'axe longitudinal.
